# EUROPEAN PATENT SPECIFICATION

(11) **EP 1 088 536 B1**
(45) Date of publication and mention of the grant of the patent: **09.03.2005**
(21) Application number: 00308474.6
(22) Date of filing: 27.09.2000
(51) Int. Cl.: A61F 13/15

(54) **A hygienic napkin**
Damenbinde
Serviette hygienique

(30) Priority: 28.09.1999 BR 9904370
(43) Date of publication of application: 04.04.2001
(73) Proprietor: Johnson & Johnson Industrial Ltda., CEP 12237-350 Sao Paulo (BR)
(72) Inventor: Carvalho, Antonio Carlos Ribeiro, Taubate, Sao Paulo, SP (BR)
(74) Representative: Mercer, Christopher Paul

(56) References cited:
- EP-A- 0 137 644
- EP-A- 0 888 765
- US-A- 5 591 150

## Description

### Disclosure of the Invention

The present invention is related to a women's disposable hygienic napkin for external use, more specifically a women's disposable hygienic napkin provided with corrugations conformable in its whole structure, which makes it possible to adapt the hygienic napkin to the user's panties according to her needs.

Women's disposable hygienic napkins are articles widely known in the state of the art that comprise an absorbent core sandwiched between two layers known as top sheet and lining sheet.

Usually, the top sheet is permeable to fluids and its function is to allow the passage of the menstrual fluid towards the absorbent core, thus keeping the fluid away from the user's body. It can be comprised of a layer of a material other than a fabric or composed of a perforated plastic film or any other material for performing said function.

Usually, the lining sheet is impermeable or resistant to the passage of liquid and is used to prevent the fluid absorbed by the absorbent core from leaking. It may comprise a non-perforated plastic sheet or any other material having such features.

The absorbent core is composed of synthetic cellulosic or textile fibers such as rayon, polyester and the like. Its purpose is to contain the vaginal exudates. In the assumption that the absorbent core to contains a superabsorbent material, it can present an large range of particle size and be distributed in any of a number of ways, for example, as a layer, or film, or as individual particles. As example of superabsorbent material that can be used is sodium polymethacrylate or any other suitable superabsorbent material.

The required features in a women's disposable hygienic napkin so that it pleases its users mainly include the anatomical adaptation, the fast absorption, the fixation in its place during use, and also must be discreet and comfortable.

The conventional women's disposable hygienic napkins shrink and the user's movement changes its initial configuration, for the movement tends to conform the napkin to the geometry of the space between the user's thighs, thus making it possible the occurrence of discomfort and premature leakage.

The prior art has tried to optimize the efficiency of those disposable product by using static concepts of the body shape in the pulp and improving the absorption of the menstrual fluid.

Examples of articles of the state of the article the purpose of which is the anatomical adaptation include the hourglass shape used in women's hygienic napkins, for example in U.S. Patent No. 3,805,790 (Kimberly-Clark) and U.S. Patent No. 4,758,241 (Elissa D. Papajohn). Said configuration takes into account the space between the user's thighs close to the vaginal region, thus providing an improvement over rectangular-shaped articles, but said women's napkins do not take into account the individuality of the space between the user's thighs and the variety of panties used nowadays.

Another important example of the prior art is the women's hygienic napkin with a corrugated absorbent core, for example in U.S. Patent No. 3,411,504 (J. A. Glasman). Said women's hygienic napkin comprises an absorbent core provided with longitudinal embossed lines for a faster flow of the menstrual fluid, but it does not take into account the user's anatomy.

Still another example of the prior art women's hygienic napkin is U.S. Patent No. 3,954,107 (Colgate Palmolive) that is provided with two parallel longitudinal absorbent cores having a channel therebetween whose function is to collect the fluid and direct same to the longitudinal ends of the article, not aiming at the women anatomy or comfort.

EP-A-0137644 provides an absorbent product comprising a corrugated fibrous webs which contains at least about 10% by weight of superabsorbent, said corrugated web being stabilized to retain its transverse folds even when wet. The stabilization is described as being achieved by placing a layer of a stabilizing substance on one side of the product over the surface at the peaks of the transverse folds, or by adding a minor portion of fibers having a lower melting point than the remaining fibers in the fibrous web and subjecting the corrugated web to temperatures sufficient to melt the fibers, thereby providing heat bonding between the folds or within the corrugations.

The objective of the present invention is to overcome the above described inconveniences by improving the state of art in order to offer the user a better adaptation, an efficient absorption, protection, comfort and discretion.

The invention is based on a women's disposable hygienic napkin for external use as defined in the appendant claims.
It should be understood by conformable corrugations the longitudinal folds throughout the structure of the absorbent article that provide a corrugated structure that is adaptable according to the user's needs at the time she puts the hygienic napkin on. The corrugations are disposed in such a way that they are parallel to the longitudinal axis of the product before use. The corrugations are not necessarily regular.

It should be understood by adaptation all and any extension or side compression against the women's hygienic napkin, in a way that said hygienic napkin can be extended and positioned by the user in the required place when she puts it on. The extensibility of the hygienic napkin attained by the corrugations provides a better adequacy to the dimensions of the panties, thus providing comfort and safety to the user.

An alternative of the present invention relates to a conformable corrugated absorbent structure that makes out the women's hygienic napkin, wherein the longitudinal folds are conformed in such a way that they are kept at least until the moment of use. The conformation of the structure of the absorbent article is stabilized by one of the sides of the product, that is, on the surface, in the lower part of the longitudinal cavities or on the inner walls of the corrugations on one of its sides.

The conformation must be kept at least until the time the napkin is put on, in such a way that the structure of the absorbent article can be adapted to the user's needs, that is, said conformation must provide a lateral extensibility to the structure of the absorbent article whenever required.

The conformation process is selected in accordance with the types of materials of the absorbent structure used. The conformation can be attained, for example, through heating, adhesive application, embossing of the tips by means of heat, adhesive, or both, and the like.

In the event of conformation through the application of an adhesive, a suitable adhesive is the pressure sensitive adhesive (PSA).

A suitable way to maintain the structure of the absorbent article is to spray an adhesive on a smooth surface, for example a film, and then overtap the structure of the previously corrugated hygienic napkin over said film.

At the time it is put on, the smooth surface is removed, the ends are extended and/or compressed and then attached to the panties.

Another typical way to conform the structure of the absorbent article is to apply adhesive lines in the inner regions of the protuberances of the corrugations and to place the corrugations close to one another.

At the time it is put on, the ends of the absorbent article are extended and/or compressed, thus exposing the adhesive regions for attaching said napkin to the user's panties.

The corrugation or longitudinal fold of the absorbent structure is attained by procedures known to one skilled in the art, for example, the use of a male-female configuration.

The absorbent article of the present invention may also comprise side fastening wings.

The present invention will be described below through the accompanying drawings that represent a preferred embodiment, without excluding other alternative embodiments of protection defined in the attached claims. The relevant figures are as follows:
Figure 1 - a perspective view of the women's hygienic napkin in accordance with the present invention;
Figure 2 - a cross-sectional cut view of the women's hygienic napkin illustrated in Figure 1;
Figure 3 - a cross-sectional cut view of a variation of the conformation of the women's hygienic napkin illustrated in Figure 1;
Figure 4 - a detailed cross-section cut view of the variation of the conformation illustrated in Figure 3, with the corrugations close to one another;
Figure 5 - a view of the women's hygienic napkin of the invention, when it is initially adjusted to the panties;
Figure 6 - a view of the women's hygienic napkin of the invention, in the sequence of the adjustment to the panties;
Figure 7 - a cross-section cut view of the women's hygienic napkin illustrated in Figure 1.

The women's hygienic napkin 10 of the present invention is comprised of a substantially oblong absorbent core 25 disposed between two layers known as top sheet 20 and lining sheet 30.

The top sheet 20 is permeable to fluid and its function is to allow the passage of the menstrual fluid towards the absorbent core 25, thus keeping the fluid away from the user's body. Therefore, it should comprise a layer of a material other than fabric or made of a perforated plastic film or any material that performs this function.

The lining sheet 30, opposite the top sheet 20, is impermeable or resistant to the passage of liquids and used to prevent the fluid absorbed by the absorbent core 25 from leaking. Therefore, it should comprise a non-perforated plastic sheet or any other material having such features.

Still according to the present invention, the women's hygienic napkin 10 is preferably provided with wings or borders 40 that if extend laterally from the top sheet 20 and/or of the lining sheet 30 of the women's hygienic napkin 10 and they help in the attachment of the napkin to the user's panties. In the lower part of each side wing, there is an adhesive 60 covered by a removable protection sheet 50.

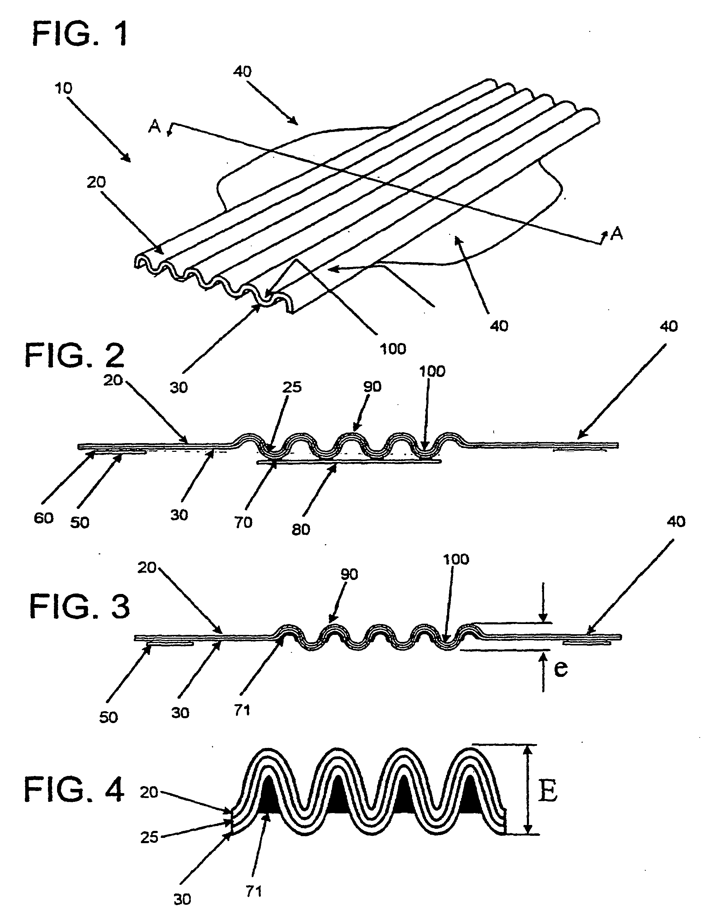

As shown in Figure 2, the women's hygienic napkin 10 is provided with longitudinal conformable corrugations that can extend throughout its whole structure. Such corrugations are formed by alternating protuberances 90 and cavities 100. Said protuberances 90 and cavities 100 are not necessarily regular.

Said corrugated structure of the women's hygienic napkin 10 can be maintained until the time of use by the adhesive regions 70 located in the lower part of the cavities 100, protected by a removable protection sheet 80.

Figure 3 shows another alternative of the stabilization of the corrugated structure of the women's hygienic napkin 10, wherein said structure is maintained through the application of adhesive 71 to the lower part of the protuberances 90, and the corrugations are disposed close to one another as shown in Figure 4. In Figure 3, it is also shown an apparent thickness "e" characterized by the formation of the corrugations. The same happens in Figure 4, wherein the apparent thickness "E" is formed by the approximation of the corrugations. Preferable, "E" up to fifteen times higher than "e".

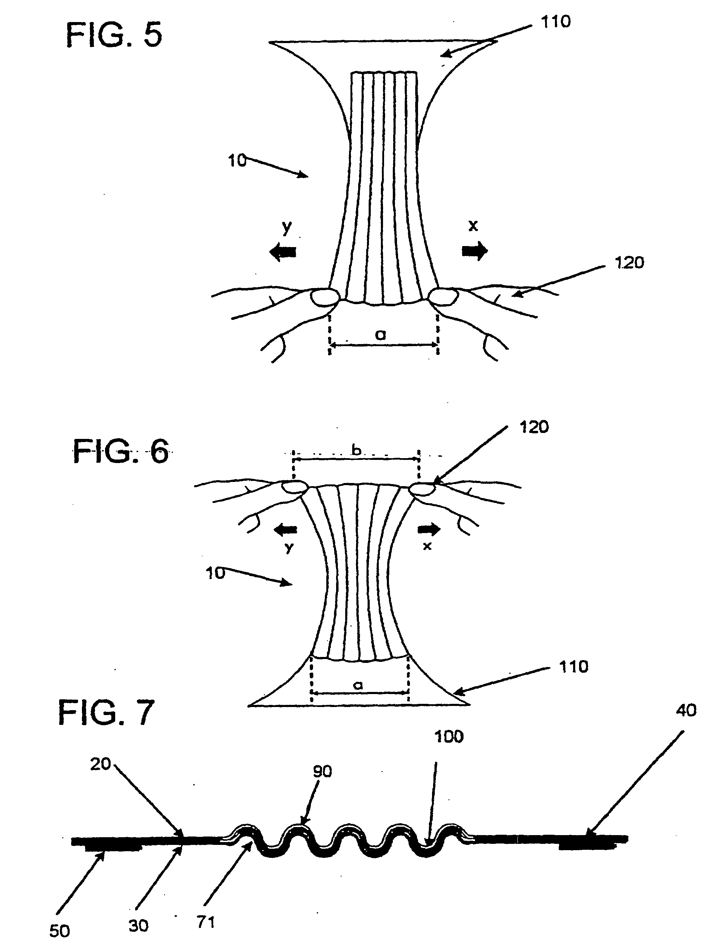

Figure 5 shows said women's hygienic napkin 10 at the time it is placed on the panties 110 of the user 120. Said napkin can be extended in the front portion in direction X as far as a position "a" according to the needs of the user 120.

Similarly, in Figure 6 the women's hygienic napkin 10 is shown extended in the lower part, in direction X as far as a position "b" according to the needs of the user 120.

Preferably, the positions "a" and "b" can be extended as far as about five times the initial width of the corrugated absorbent.

Figure 7 shows another alternative of the stabilization of the corrugated structure of the hygienic napkin 10, wherein said structure is maintained through the application of adhesive 71 to the lower part of the protuberances 90 and cavities 100 continuously.

Although not shown in the accompanying figures, the central third part of the absorbent article 10 can be made of a non-corrugated material.

## Claims

1. A disposable women's hygienic napkin (10) for external use, composed of longitudinal conformable corrugations comprising corrugated absorbent (20, 25, 30) in its whole structure that allow the hygienic napkin (10) to extend and/or shrink to adapt the same to the user's panties.

2. A disposable women's hygienic napkin (10) according to claim 1, wherein said extension (a,b) of the corrugated absorbent (20, 25, 30) is up to about five times its initial width.

3. A disposable women's hygienic napkin (10) according to claim 1, wherein the thickness (E) of the corrugated absorbent when fully compressed is up to about fifteen times the initial thickness (e) of the corrugated absorbent.

4. A disposable women's hygienic napkin (10) according to claim 1, also comprising wings (40) that extend laterally.

5. A disposable women's hygienic napkin (10) according to claim 1, wherein, before use, the corrugations are maintained bya flat surface (80) that contacts the adhesive regions (70) in the lower parts of the cavities (100).

6. A disposable women's hygienic napkin (10) according to claim 1, wherein, before use, the corrugations are maintained by the adhesive lines (71) disposed between the panty facing regions of the protuberances (90) of the corrugations.

7. A disposable women's hygienic napkin (10) according to claim 1, wherein, before use, the corrugations are maintained by the adhesive lines (71) disposed between the the panty facing regions of the protuberances (90) and also within the cavities (100) the corrugations.

8. A disposable women's hygienic napkin (10) for external use, composed of longitudinal conformable corrugations comprising corrugated absorbent (20, 25, 30) in its whole structure apart from the central third that allow the hygienic napkin (10) to extend and/or shrink to adapting same to the user's panties, wherein the central third of the women's hygienic napkin is non-corrugated.

## Patentansprüche

1. Einweg-Damenbinde (10) für den äußeren Gebrauch bestehend aus einer gleichförmigen Längs-Wellenanordnung aus gewelltem Absorptionsmittel (20, 25, 30) in ihrer gesamten Struktur, welche es ermöglicht die Damenbinde (10) zur Anpassung an den Schlüpfer der Trägerin zu dehnen und/oder zu stauchen.

2. Einweg-Damenbinde (10) nach Anspruch 1, bei welcher die Dehnung (a, b) des gewellten Absorptionsmittels (20, 25, 30) bis zum Fünffachen der anfänglichen Breite beträgt.

3. Einweg-Damenbinde (10) nach Anspruch 1, bei welcher die Dicke (E) des gewellten Absorptionsmittels nach dem vollständigen Komprimieren bis zum Fünfzehnfachen der anfänglichen Dicke (e) des gewellten Absorptionsmittels beträgt.

4. Einweg-Damenbinde (10) nach Anspruch 1, welche auch sich seitlich erstreckende Flügel (40) aufweist.

5. Einweg-Damenbinde (10) nach Anspruch 1, bei welcher die Wellenanordnung vor dem Gebrauch durch eine ebene Fläche (80) gehalten wird, welche Klebebereiche (70) in den unteren Teilen der Vertiefungen (100) berührt.

6. Einweg-Damenbinde (10) nach Anspruch 1, bei welcher die Wellenanordnung vor dem Gebrauch durch Klebelinien (71) gehalten wird, welche zwischen den dem Schlüpfer zugewandten Bereichen der Vorsprünge (90) der Wellenanordnung angeordnet sind.

7. Einweg-Damenbinde (10) nach Anspruch 1, bei welcher die Wellenanordnung vor dem Gebrauch durch Klebelinien (71) gehalten wird, welche zwischen den dem Schlüpfer zugewandten Bereichen der Vorsprünge (90) und auch in den Vertiefungen (100) der Wellenanordnung angeordnet sind.

8. Einweg-Damenbinde (10) für den äußeren Gebrauch bestehend aus einer gleichförmigen Längs-Wellenanordnung aus gewelltem Absorptionsmittel (20, 25, 30) in ihrer gesamten Struktur außerhalb des zentralen Drittels, welche es ermöglicht die Damenbinde (10) zur Anpassung an den Schlüpfer der Trägerin zu dehnen und/oder zu stauchen, wobei das zentrale Drittel der Damenbinde nicht gewellt ist.

## Revendications

1. Serviette hygiénique jetable (10) pour usage externe, composée d'ondulations longitudinales adaptables comprenant un absorbant ondulé (20, 25, 30) dans la totalité de sa structure, qui permettent à la serviette hygiénique (10) de s'étendre et/ou de rétrécir pour s'adapter à la culotte de l'utilisatrice.

2. Serviette hygiénique jetable (10) selon la revendication 1, dans laquelle ladite extension (a, b) de l'absorbant ondulé (20, 25, 30) atteint environ cinq fois sa largeur initiale.

3. Serviette hygiénique jetable (10) selon la revendication 1, dans laquelle l'épaisseur (E) de l'absorbant ondulé lorsqu'il est complètement comprimé atteint environ quinze fois l'épaisseur initiale (e) de l'absorbant ondulé.

4. Serviette hygiénique jetable (10) selon la revendication 1, comprenant en outre des ailettes (40) qui s'étendent de manière latérale.

5. Serviette hygiénique jetable (10) selon la revendication 1, dans laquelle, avant utilisation, les ondulations sont soutenues par une surface plate (80) qui entre en contact avec les zones adhésives (70) situées dans les parties inférieures des cavités (100).

6. Serviette hygiénique jetable (10) selon la revendication 1, dans laquelle, avant utilisation, les ondulations sont soutenues par les lignes adhésives (71) disposées entre les zones des protubérances (90) des ondulations faisant face à la culotte.

7. Serviette hygiénique jetable (10) selon la revendication 1, dans laquelle, avant utilisation, les ondulations sont soutenues par les lignes adhésives (71) disposées entre les zones des protubérances (90) faisant face à la culotte, et également à l'intérieur des cavités (100) des protubérances.

8. Serviette hygiénique jetable (10) pour usage externe, composée d'ondulations longitudinales adaptables comprenant un absorbant ondulé (20, 25, 30) dans la totalité de sa structure à l'exception du tiers central, permettant à la serviette hygiénique (10) de s'étendre et/ou de rétrécir pour s'adapter à taille exacte de la culotte de l'utilisatrice, dans laquelle le tiers central de la serviette hygiénique n'est pas ondulé.
